# EUROPEAN PATENT SPECIFICATION

(11) **EP 2 254 890 B1**
(45) Date of publication and mention of the grant of the patent: **08.04.2015**
(21) Application number: 08709117.9
(22) Date of filing: 20.02.2008
(51) Int. Cl.: C07D 475/04, A61K 31/519

(54) **PROCESS FOR THE DIASTEREOISOMERIC RESOLUTION OF 5-METHYLTETRAHYDROFOLIC ACID**
VERFAHREN ZUR DIASTEREOISOMEREN RAZEMATSPALTUNG VON 5-METHYLTETRAHYDROFOLSÄURE
PROCÉDÉ DE RÉSOLUTION DIASTÉRÉOISOMÉRIQUE D'ACIDE 5-MÉTHYLTÉTRAHYDROFOLIQUE

(43) Date of publication of application: 01.12.2010
(73) Proprietor: Gnosis S.p.A., 20033 Desio (MI) (IT)
(72) Inventor: VALOTI, Ermanno, I-20033 Desio (MI) (IT); BIANCHI, Davide, I-20033 Desio (MI) (IT); VALETTI, Marco, I-20033 Desio (MI) (IT)
(74) Representative: Mancini, Vincenzo
(86) International application number: PCT/EP2008/052034
(87) International publication number: WO 2009/103333

(56) References cited:
- WO-A-2008/031284
- US-A- 5 194 611
- US-A- 5 457 202

## Description

The present invention relates to a process for the diastereoisomeric resolution of 5-methyltetrahydrofolic acid. The invention discloses also a crystalline salt of 5-methyltetrahydrofolic acid and is further directed to a process for the transformation of the single diastereoisomers of the 5-methyltetrahydrofolic salts obtained by the process of the invention into the corresponding free acid or into an alkaline earth metal salt. 5-Methyltetrahydrofolic acid, i.e. (6R,S)-N-[4-[[(2-amino-1,4,5,6,7,8-hexahydro-methyl-4-oxo-5-methyl-pteridinyl)methyl] amino]benzoyl]-L-glutamic acid -hereinafter 5-MTHF- and its derivatives have two asymmetric centres, the one belonging to the L-glutamic acid moiety and a second chiral centre on the carbon in position 6 of the pteridinyl moiety.

The configuration of the natural isomer corresponds to (6S)-N-[4-[[(2-amino-1,4,5,6,7,8-hexahydro-5-methyl-4-oxo-pteridinyl)methyl]amino]benzoyl]-L-glutamic acid, having the following formula:

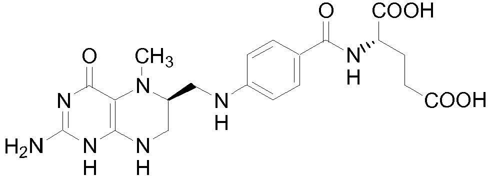

This natural diastereoisomer, found commonly in foods, is the essential endogenous form utilised and stored in the human body whereas the unnatural diastereoisomer is therapeutically ineffective since it is poorly and slowly metabolized and therefore excreted as it is. It is accordingly self-evident the need to administer the natural diastereoisomer. Nevertheless, in view of the difficulties to effectively separate the diastereoisomers, 5-methyltetrahydrofolic acid is used both as a diastereomer mixture and as a natural isomer, mainly in the form of calcium salts, as pharmaceutical or nutraceutical for the treatment of -for instance-megaloblastic folic acid anaemia, as an antidote for improving the tolerance of folic acid antagonists, specifically aminopterin and methotrexate in cancer therapy ("antifolate rescue"), for enhancing the therapeutic effect of fluorinated pyrimidines and for the treatment of autoimmune diseases such as psoriasis and rheumatic arthritis, for improving the tolerance of certain antiparasitics, such as trimethoprim-sulfamethoxazole, and for reducing the toxicity of dideazatetrahydrofolates in chemotherapy.

In order to either synthesize or isolate the natural diastereoisomer of various derivatives of folic acid, either substituted or reduced, among which 5-MTHF is likely to be the most studied, a number of methods has been disclosed in the last decades.

Given the amphoteric nature of these derivatives of folic acid and also in view of the fact that the isomers to be separated are already diastereoisomers, these resolution methods can be basically classified depending on whether they take advantage of the acid or basic characteristics of such derivatives or of the different solubility of the corresponding tetrahydrofolates as free acids.

Among the processes exploiting the acid characteristics of folic acid derivatives, EP 0537842 discloses -*inter alia*- a process for the separation of 6(R,S)-5-MTHF and 6(R,S)-5-formyltetrahydrofolic acid by precipitating -in water- a mixture of inorganic salts thereof, preferably alkali metal or alkaline earth metal salts, and subsequent treatment with a strong acid, finally adjusting the pH close to neutrality. Further, US 5,194,611 and US 5,382,581 disclosing salts of N⁵-methyl-5,6,7,8 tetrahydrofolic acid with cyclohexyl amine, diisopropyl amine, benzyl amine, ammonia, ethanol amine, triethanol amine, 2-dimethyl amino-ethanol, tert-butyl amine, lysine, or arginine, describe a process for the preparation and separation of such salts, in the presence of a mixture of solvents containing water and at least one totally water miscible solvent, -the solvent being preferably in an amount of 50-95%-, into the single (6R)- and (6S)-diastereoisomers, the latter being possibly transformed into the corresponding alkali metal or alkaline earth metal salts.

US 5,457,202 relates to a process for the resolution of (6R,S)-5-methyltetrahydrofolic acid comprising taking up the acid or a water-soluble salt thereof in water and adding a solution of N-ethyl-2-aminomethylpyrrolidine or an optical isomer thereof in water, subsequently precipitating the resulting salt. Heating the reaction mixture to 40-90°C, results in the salt of the 6S-acid dissolution, which is thus separated from the salt of the 6R-acid. The salt of the 6S-acid precipitates again on cooling the solution. Suspension of the latter in water and addition of sodium hydroxide solution results in the sodium salt that can be converted with, for example, an alkaline earth metal hydroxide or chloride into an alkaline earth metal salt such as the calcium salt. The yields of (6S)-5-MTHF are however poor, also in view of the repeated crystallizations required, and the process does not represent a commercially and industrially effective alternative.

IT 1254954 discloses a process for preparing the (6S) diastereoisomer of folinic acid comprising, in the presence of organic solvents, preferably methylethyl ketone and acetone, the salification of the racemic mixture of said acid or of 5-N-formyl-5,6,7,8-tetrahydropteroic acid, with S-(alpha)-methyl-p-nitro-benzylamine or (S)-(-)-alpha-phenylethylamine and the subsequent crystallization of the salt containing the optically active correspondent form.

Among the processes exploiting the basic characteristics of folic acid derivatives US 5,006,655 discloses a process for the preparation of 5,10-methenyl-(6R)-, 5-formyl-(6S)-, or 5-methyl-(6S)-tetrahydrofolic acid or a salt thereof comprising fractionally crystallizing 5,10-methenyl-(6R,S)-tetrahydrofolic acid or a salt thereof, which is present in at least a 70% by weight concentration, in the presence of organic solvents.

US 5,324,836 discloses a process for the preparation of (6S)-and (6R)-tetrahydrofolic acid and their addition salts with sulphonic acids or with sulphuric acid comprising the steps of reacting (6R,S)-tetrahydrofolic acid with a sulphonic acid or sulphuric acid and fractionally crystallizing the resulting addition salt, preferably in the presence of an oxidation inhibitor.

US 6,858,731 discloses a process for preparing and concentrating (6S,αS) or (6S,αR) tetrahydrofolic acid ester salts and (6S,αS) or (6S,αR) tetrahydrofolic acid comprising preparing or dissolving diastereoisomers of addition salts of tetrahydrofolic acid esters with aromatic sulphonic acids in organic solvents and crystallizing them.

Among the processes exploiting the different solubility of the corresponding tetrahydrofolates as free acids, US 5,489,684 discloses a process for the preparation of (6S)-5,6,7,8-tetrahydrofolic acid having a diastereoisomeric purity of at least 75% comprising the direct crystallization from an aqueous solution of an alkali metal salt of the (6R,S) diastereoisomeric mixture of 5,6,7,8-tetrahydrofolic acid added with a strong non-oxidizing acid or acetic acid at 5-80°C, adjusting the pH at 4.8-5.3.

Further, US 6,271,374 discloses crystalline (6S)-tetrahydrofolic acid having a purity higher than 98% and a process for the preparation thereof comprising the crystallization of (6S)- or (6R,S)-tetrahydrofolic acid in water or in a mixture of water and a water miscible organic solvent.

Some further resolution has been proposed in US 5,698,693 wherein a separation by chiral chromatography or fractional crystallization carried out on α-esters of (6R,S)-5,6,7,8-tetrahydrofolic acid derivatives, subsequently deprotected, is disclosed.

Chromatography in chiral phase is instead disclosed in EP 0627435 concerning the resolution of diastereoisomeric mixtures of some tetrahydrofolates.

However, either any of these processes show the need of repeated crystallizations, or they require organic solvents or mixtures thereof with water, or they show low yields or diastereoisomeric purity or expensive reagents.

Hence, it is a general object of the present invention to provide a simple, economically advantageous and efficient process, by which a mixture of (6R,S)-diastereoisomers of (6R,S)-N-[4-[[(2-amino-1,4,5,6,7,8-hexahydro-methyl-4-oxo-5-methyl-pteridinyl)methyl] amino]benzoyl]-L-glutamic acid may be separated into the corresponding stable and pure (6R) and (6S)-diastereoisomers thereof, in a good yield and diastereoisomeric purity.

According to a first aspect, the present invention relates to a process for the separation of a mixture of diastereoisomers of (6R,S)-5-methyltetrahydrofolic acid or an alkali metal salt thereof comprising the reaction of the mixture in water with at least one organic base selected from the group consisting of 1-phenylethylamine, 1-p-nitrophenylethylamine, 1-p-chlorophenylethylamine, 1-p-methylphenylethylamine, 1-p-methoxyphenylethylamine, 1-(1-naphthyl)ethylamine, 1-(2-naphthyl)ethylamine, either in a (R,S)- or (R)- or (S)-configuration, so to obtain the corresponding amine salt, and the recovering of a diastereoisomer, the latter being the corresponding natural (6S) diastereoisomer substantially pure. In particular, the process of the invention comprises that the at least one diastereoisomer is recovered by crystallization; preferably, the crystallized salt of the diastereoisomer is re-crystallized at least once.

It is also preferred that the at least one diastereoisomer is transformed into the free acid or into an alkaline earth metal salt.

According to preferred embodiments, the alkaline earth metal salt so obtained can be transformed into the free acid whereas the free acid so obtained can be transformed, in its turn, into an alkali metal or alkaline earth metal salt. The most preferred alkaline earth metal salt is a calcium salt.

The process of the invention allows to cheaply isolate the natural isomer (6S)-5-MTHF substantially pure, this generally meaning a concentration of the diastereoisomer greater than 95 wt%, preferably greater than 99 wt% and showing an extremely high diastereoisomeric purity (higher than 99%).

Further, the process of the invention is carried out in water in the total absence of any other organic solvent both during the crystallization of the amine salt and the recovering of the free acid from such salt.

Another aspect of the present invention relates to a compound which is a crystalline salt of (6S)- 5-methyltetrahydrofolic acid with at least one organic base selected from the group consisting of 1-phenylethylamine, 1-p-nitrophenylethylamine, 1-p-chlorophenylethylamine, 1-p-methylphenylethylamine, 1-p-methoxyphenylethylamine, 1-(1-naphthyl)ethylamine, 1-(2-naphthyl)ethylamine, either in a (R,S)- or (R)- or (S)-configuration.

In particular, the compound of the invention is selected from the group consisting of (R,S)-1-phenylethylamine 5-methyl-(6S)-tetrahydrofolate; (R)-1-phenylethylamine 5-methyl-(6S)-tetrahydrofolate; (S)-1-phenylethylamine 5-methyl-(6S)-tetrahydrofolate.

The 5-methyltetrahydrofolic moiety of the compound of the invention is preferably in a (6S) configuration.

Still more preferred compounds of the invention are selected from the group consisting of (R)-, (S)- and (R,S)-1-phenylethylamine 5-methyl-(6S)-tetrahydrofolate.

In particular, (R)-, (S)- and (R,S)-1-phenylethylamine 5-methyl-(6S)-tetrahydrofolate are the most preferred compounds of the invention.

It can be advantageously noted that the crystalline amine salts of the invention show a high chemical stability.

The use of the compound of the invention for the separation of a mixture of diastereoisomers of (6R,S)-5-methyltetrahydrofolic acid or an alkali metal salt thereof is still another aspect of the present invention as recited in claim 10.

The compound of the invention shows an extraordinary long lasting chemical stability, this actually guaranteeing quite unaltered purity, even after months, and that the titre of the correspondent 5-MTHF moiety results substantially unchanged.

The compound of the invention can be prepared by simply applying the common general knowledge of the field, as the skilled man would easily understand; however, as a non-limitative example, the compound of the invention can be obtained by filtration of the precipitate obtained adding the (6R,S)-5-methyltetrahydrofolic acid to an aqueous solution containing (R)-, (S)- and (R,S)-1-phenylethylamine, (or vice versa) preferably maintained under nitrogen and stirring the base being in a molar amount of about 200 to 300% of the acid. The following examples illustrate the invention without limiting it.

### Example 1

### Preparation of (R,S)-1-phenylethylamine 5-methyl-(6S)-tetrahydrofolate dihydrate

Under nitrogen, 13.2 g (109 mmol) of (R,S)-1-phenylethylamine were added to a suspension of 20 g (42 mmol) of 5-methyl-(6R,S)-tetrahydrofolic acid monohydrate in water (100 ml) and heated to 60°C for 2 h. The solution was cooled to room temperature and stirred for 72 h. After cooling to 0°C for 4 h, the suspension was filtered under suction and washed with water (2 x 15 ml). After drying at 40°C for 4 h, 13.4 g of crystalline salt of the title product was obtained which showed a (6S)/(6R) diastereoisomer ratio of 93.3/6.7 (see analytical data).
HPLC data: a) titre as a mixture of 5-methyl-(6S)-tetrahydrofolic acid and 5-methyl-(6R)-tetrahydrofolic acid: calculated 65.5% (on the dry product); found 64.51 (98.5 % of the theoretical value); b) diastereoisomer composition (HAS chiral): 5-methyl-(6S)-tetrahydrofolic acid 93.3%; 5-methyl-(6R)-tetrahydrofolic acid 6.7%;
Specific rotation [α]²⁰_{D} = +31.38° (C = 1 in water)
NMR (DMSO-_{d6})ppm 7.75 (d*, H) ; 7.57 (d, 2H) ; 7.4 (d, 4H) ; 7.30 (t, 4H), 7.25 (d, 2H); 6.57 (d, 2H), 6.42 (bs 2H); 6.60-5.80 (m* 11H) ; 4.08 (m, 3H) ; 3.18 (bd, 2H); 2.95-2.70 (m 3H) ; 2.45 (s, 3H); 2.10 (m, 2H); 1.85 (m, 2H); 1.37 (d, 6H). *The signals exchange with D₂O.

### Example 2

### Preparation of (R)-1-phenylethylamine 5-methyl-(6S)-tetrahydrofolate dehydrate.

Under nitrogen, 13.2 g (109 mmol) of (R)-1-phenylethylamine were added to a suspension of 20 g (42 mmol) of 5-methyl-(6R,S)-tetrahydrofolic acid monohydrate in water (100 ml) and heated to 60°C for 2 h. The solution was cooled to room temperature and stirred for 72 h. After cooling to 0°C for 4 h, the suspension was filtered under suction and washed with water (2 x 15 ml). After drying at 40°C for 4 h, 14.2 g of crystalline salt of the title product were obtained showing a (6S)/(6R) diastereoisomer ratio of 91.1/8.9 (see analytical data).
HPLC data: a) titre as a mixture of 5-methyl-(6S)-tetrahydrofolic acid and 5-methyl-(6R)-tetrahydrofolic acid: calculated 65.5% (on the dry product); found 64.75 (98.9 % of the theoretical value); b) diastereoisomer composition (HAS chiral): 5-methyl-(6S)-tetrahydrofolic acid 91.1%; 5-methyl-(6R)-tetrahydrofolic acid 8.9%;
DSC two endotherms at 185 (eutectic peak) and 239°C (melting point) .
Specific rotation [α]²⁰_{D} = +33.4 (C = 1 in water)
NMR (DMSO-_{d6})ppm 7.75 (d*, H); 7.57 (d, 2H); 7.4 (d, 4H); 7.30 (t, 4H), 7.25 (d, 2H); 6.57 (d, 2H), 6.42 (bs 2H); 6.60-5.80 (m* 11H) ; 4.08 (m, 3H); 3.18 (bd, 2H); 2.95-2.70 (m 3H); 2.45 (s, 3H); 2.10 (m, 2H); 1.85 (m, 2H); 1.37 (d, 6H). *The signals exchange with D₂O.

### Example 3

### Preparation of (S)-1-phenylethylamine 5-methyl-(6S)-tetrahydrofolate dihydrate

Under nitrogen, 13.2 g (109 mmol) of (S)-1-phenylethylamine were added to a suspension of 20 g (42 mmol) of 5-methyl-(6R,S)-tetrahydrofolic acid monohydrate in water (100 ml) and heated to 60°C for 2h. The solution was cooled to room temperature and stirred for 72 h. After cooling to 0°C for 4 h, the suspension was filtered under suction and washed with water (2 x 15 ml). After drying at 40°C for 4 h, 13.7 g of crystalline salt of the title product were obtained showing a (6S)/(6R) diastereoisomer ratio of 95.8/4.2 (see analytical data).
HPLC data: a) titre as a mixture of 5-methyl-(6S)-tetrahydrofolic acid and 5-methyl-(6R)-tetrahydrofolic acid: calculated 65.5% (on the dry product); found 64.3 (98.2% of the theoretical value); b) diastereoisomer composition (HAS chiral): 5-methyl-(6S)-tetrahydrofolic acid 95.8%; 5-methyl-(6R)-tetrahydrofolic acid 4.2%;
DSC: two endotherms at 183 (eutectic peak) and 245°C (melting point)
Specific rotation [α]²⁰_{D} = +28.8 (C = 1 in water)
NMR (DMSO-_{d6})ppm 7.75 (d*, H) ; 7.57 (d, 2H) ; 7.4 (d, 4H) ; 7.30 (t, 4H), 7.25 (d, 2H) ; 6.57 (d, 2H), 6.42 (bs 2H) ; 6.60-5.80 (m* 11H) ; 4.08 (m, 3H) ; 3.18 (bd, 2H); 2.95-2.70 (m 3H); 2.45 (s, 3H); 2.10 (m, 2H); 1.85 (m, 2H); 1.37 (d, 6H). *The signals exchange with D₂O.

### Example 4

### Crystallisation of (S)-1-phenylethylamine 5-methyl-(6S)-tetrahydrofolate dihydrate

Under nitrogen, a suspension of 13.2 g (18.8 mmol) of crystalline product, prepared according to Example 3, in water (66 ml) was heated to 60°C for 1 h and then cooled at room temperature for 4h. This procedure was performed four times. After cooling to 0° for 4 h, the suspension was filtered under suction and washed with water (2 x 10 ml). After drying at 40°C for 4 h, 10.9 g of crystalline salt of the title product was obtained which showed a (6S)/(6R) diastereoisomer ratio of 99.2/0.8 (see analytical data).
HPLC data: a) titre as a mixture of 5-methyl-(6S)-tetrahydrofolic acid and 5-methyl-(6R)-tetrahydrofolic acid: calculated 65.5% (on the dry product); found 64.9 (99.1% of the theoretical value); b) diastereoisomer composition (HAS chiral): 5-methyl-(6S)-tetrahydrofolic acid 99.2%; 5-methyl-(6R)-tetrahydrofolic acid 0.8%;
DSC: one endotherm at 248.1°C (melting point).
Specific rotation [α]²⁰_{D} = +28.7 (C = 1 in water).

### Example 5

### Preparation of 5-methyl-(6S)-tetrahydrofolic acid.

Under nitrogen, a suspension of 10.9 g (15.14 mmol) of crystalline product, prepared according to Example 3, in water (66 ml) was acidified with 10% HCl until pH 3. After cooling to 0°C for 4 h, the suspension was filtered under suction and washed with water (2 x 10 ml). After drying at 40°C for 4 h, 6.9 g of 5-methyl-(6S)-tetrahydrofolic acid was obtained. HPLC data: a) titre as a mixture of 5-methyl-(6S)-tetrahydrofolic acid and 5-methyl-(6R)-tetrahydrofolic acid: calculated 100%; found 99.3; b) diastereoisomer composition (HAS chiral): 5-methyl-(6S)-tetrahydrofolic acid 99.5%; 5-methyl-(6R)-tetrahydrofolic acid 0.5%;
DSC: endotherm at 256.2 °C.
Specific rotation [α]²⁰_{D} = +14.3 (C = 1 in NaOH 0.01N).

### Example 6

### Preparation of 5-methyl-(6R,S)-tetrahydrofolic acid.

Under nitrogen, a suspension of 30 g of amorphous 5-methyl-(6R,S)-tetrahydrofolic acid calcium salt, corresponding to 25.5 g of anhydrous compound (51 mmol), in water (66 ml) was acidified with 10% HCl until pH 3. After cooling to 0°C for 4 h, the suspension was filtered under suction and washed with water (2 x 10 ml). After drying at 40°C for 4 h, 24.0 g of 5-methyl-(6R,S)-tetrahydrofolic acid was obtained.
HPLC data: a) titre as a mixture of 5-methyl-(6S)- and 5-methyl-(6R)-tetrahydrofolic acid: calculated 100%; found 99.3; b) diastereoisomer composition (HAS chiral): 5-methyl-(6S)-tetrahydrofolic acid 50.5%; 5-methyl-(6R)-tetrahydrofolic acid 49.5%;
DSC: endotherm at 248.3 °C.
Specific rotation [α]²⁰_{D} = +19.2 (C = 1 in NaOH 0.01N).

### STABILITY

The stability of the compounds listed in the following table, under powder form and in sealed aluminium foil bag, was tested, keeping the samples in airtight container protected from light and measuring the purity and the titre thereof after 6 and 12 months.

| Compound | Starting values | | After 6 months | | After 12 months | |
|---|---|---|---|---|---|---|
| | Purity | Titre | Purity | Titre | Purity | Titre |
| (R)-PEA (6S)-5-MTHF | 99.8 | 64.5 | 99.3 | 64.35 | 99.0 | 64.1 |
| (S)-PEA (6S)-5-MTHF | 99.7 | 64.3 | 99.6 | 64.2 | 99.5 | 64.0 |

An expiration date of 24 months could be safely established for the compound of the invention. Any of the compounds listed in the above table showed a high stability since both their purity and titre, even after 12 months, resulted to fall well within the required specifications. In particular, the stability of the acid moiety was observed by HPLC, detecting the purity and titre thereof.

## Claims

1. A process for the separation of a mixture of diastereoisomers of (6R,S)-5-methyltetrahydrofolic acid or an alkali metal salt thereof comprising the reaction of the mixture in water with at least one organic base selected from the group consisting of 1-phenylethylamine, 1-p-nitrophenylethylamine, 1-p-chlorophenylethylamine, 1-p-methylphenylethylamine, 1-p-methoxyphenylethylamine, 1-(1-naphthyl)ethylamine, 1-(2-naphthyl)ethylamine, either in a (R,S)- or (R)- or (S)- configuration, so to obtain the corresponding amine salt, and the recovering of a diastereoisomer, the latter being the corresponding natural (6S) diastereoisomer substantially pure.

2. A process according to the previous claim, comprising recovering the at least one diastereoisomer by crystallization.

3. A process according to the previous claim, wherein the crystallized salt of the diastereoisomer is re-crystallized at least once.

4. A process according to any of the previous claims, wherein the at least one diastereoisomer is transformed into the free acid or into an alkaline earth metal salt.

5. A process according to the previous claim, wherein the free acid is transformed into an alkali metal or alkaline earth metal salt.

6. A process according to claim 4 or 5, wherein the alkaline earth metal salt is a calcium salt.

7. A process according to any of claims 4-6, wherein the alkaline earth metal salt is transformed into the free acid.

8. A crystalline compound which is a salt of (6S)- 5-methyltetrahydrofolic acid with at least one organic base selected from the group consisting of 1-phenylethylamine, 1-p-nitrophenylethylamine, 1-p-chlorophenylethylamine, 1-p-methylphenylethylamine, 1-p-methoxyphenylethylamine, 1-(1-naphthyl)ethylamine, 1-(2-naphthyl)ethylamine, either in a (R,S)- or (R)- or (S)- configuration.

9. The compound according to the previous claim, which is selected from the group consisting of (R,S)-1-phenylethylamine 5-methyl-(6S)-tetrahydrofolate; (R)-1-phenylethylamine 5-methyl-(6S)-tetrahydrofolate; (S)-1-phenylethylamine 5-methyl-(6S)-tetrahydrofolate.

10. Use of the compound according to any of claims 8-9, for the separation of a mixture of diastereoisomers of (6R,S)-5-methyltetrahydrofolic acid or an alkali metal salt thereof comprising the reaction of the mixture in water with at least one organic base selected from the group consisting of 1-phenylethylamine, 1-p-nitrophenylethylamine, 1-p-chlorophenylethylamine, 1-p-methylphenylethylamine, 1-p-methoxyphenylethylamine, 1-(1-naphthyl)ethylamine, 1-(2-naphthyl)ethylamine, either in a (R,S)- or (R)- or (S)- configuration, so to obtain the corresponding amine salt, and the recovering of a diastereoisomer, the latter being the corresponding natural (6S) diastereoisomer substantially pure.

## Patentansprüche

1. Verfahren für die Auftrennung eines Gemisches von Diastereoisomeren von (6R,S)-5-Methyltetrahydrofolsäure oder einem Alkalimetallsalz davon, umfassend die Reaktion des Gemisches in Wasser mit wenigstens einer organischen Base, ausgewählt aus der Gruppe, bestehend aus 1-Phenylethylamin, 1-p-Nitrophenylethylamin, 1-p-Chlorphenylethylamin, 1-p-Methylphenylethylamin, 1-p-Methoxyphenylethylamin, 1-(1-Naphthyl)ethylamin, 1-(2-Naphthyl)ethylamin, entweder in (R,S)- oder (R)- oder (S)-Konfiguration, um so das entsprechende Aminsalz zu erhalten, und Gewinnung eines Diastereoisomers, wobei das letztgenannte das entsprechende natürliche (6S)-Diastereoiso8mer ist, das im Wesentlichen rein ist.

2. Verfahren gemäß dem vorangehenden Anspruch, das Gewinnen wenigstens eines Diastereoisomers durch Kristallisation umfasst.

3. Verfahren gemäß dem vorangehenden Anspruch, wobei das kristallisierte Salz des Diastereoisomers wenigstens einmal umkristallisiert wird.

4. Verfahren gemäß einem der vorangehenden Ansprüche, wobei das wenigstens eine Diastereoisomer in die freie Säure oder in ein Erdalkalimetallsalz transformiert wird.

5. Verfahren gemäß dem vorangehenden Anspruch, wobei die freie Säure in ein Alkalimetall- oder Erdalkalimetallsalz transformiert wird.

6. Verfahren gemäß Anspruch 4 oder 5, wobei das Erdalkalimetallsalz ein Calciumsalz ist.

7. Verfahren gemäß einem der Ansprüche 4 bis 6, wobei das Erdalkalimetallsalz in die freie Säure transformiert wird.

8. Kristalline Verbindung, die ein Salz von (6S)-5-Methyltetrahydrofolsäure mit wenigstens einer organischen Base, ausgewählt aus der Gruppe, bestehend aus 1-Phenylethylamin, 1-p-Nitrophenylethylamin, 1-p-Chlorphenylethylamin, 1-p-Methylphenylethylamin, 1-p-Methoxyphenylethylamin, 1-(1-Naphthyl)ethylamin, 1-(2-Naphthyl)ethylamin, entweder in (R,S)- oder (R)- oder (S)-Konfiguration, ist.

9. Verbindung gemäß dem vorangehenden Anspruch, die ausgewählt ist aus der Gruppe, bestehend aus (R,S)-1-Phenylethylamin-5-methyl-(6S)-tetrahydrofolat; (R)-1-Phenylethylamin-5-methyl-(6S)-tetrahydrofolat; (S)-1-Phenylethylamin-5-methyl-(6S)-tetrahydrofolat.

10. Verwendung der Verbindung gemäß einem der Ansprüche 8 bis 9 für die Auftrennung eines Gemisches von Diastereoisomeren von (6R,S)-5-Methyltetrahydrofolsäure oder einem Alkalimetallsalz davon, umfassend die Reaktion des Gemisches in Wasser mit wenigstens einer organischen Base, ausgewählt aus der Gruppe, bestehend aus 1-Phenylethylamin, 1-p-Nitrophenylethylamin, 1-p-Chlorphenylethylamin, 1-p-Methylphenylethylamin, 1-p-Methoxyphenylethylamin, 1-(1-Naphthyl)ethylamin, 1-(2-Naphthyl)ethylamin, entweder in (R,S)- oder (R)- oder (S)-Konfiguration, um so das entsprechende Aminsalz zu erhalten, und Gewinnen eines Diastereoisomers, wobei das letztgenannte das entsprechende natürliche (6S)-Diastereoisomer ist, das im Wesentlichen rein ist.

## Revendications

1. Procédé pour la séparation d'un mélange de diastéréoisomères d'acide (6R,S)-5-méthyltétrahydropholique ou d'un sel de métal alcalin de celui-ci comprenant la réaction du mélange dans de l'eau avec au moins une base organique sélectionnée parmi le groupe constitué de 1-phényléthylamine, 1-p-nitrophényléthylamine, 1-p-chlorophényléthylamine, 1-p-méthylphényléthylamine, 1-p-méthoxyphényléthylamine, 1-(1-naphtyl)éthylamine, 1-(2-naphtyl)éthylamine, dans l'une ou l'autre configuration (R,S)- ou (R)- ou (S)-, de manière à obtenir le sel d'amine correspondant, et la récupération d'un diastéréoisomère, ce dernier étant le diastéréoisomère (6S) naturel correspondant sensiblement pur.

2. Procédé selon la revendication précédente, comprenant la récupération du au moins un diastéréoisomère par cristallisation.

3. Procédé selon la revendication précédente, dans lequel le diastéréoisomère cristallisé est recristallisé au moins une fois.

4. Procédé selon l'une quelconque des revendications précédentes, dans lequel le au moins un diastéréoisomère est transformé en l'acide libre ou en un sel de métal alcalino-terreux.

5. Procédé selon la revendication précédente, dans lequel l'acide libre est transformé en un sel de métal alcalin ou de métal alcalino-terreux.

6. Procédé selon les revendications 4 ou 5, dans lequel le sel de métal alcalino-terreux est un sel de calcium.

7. Procédé selon l'une quelconque des revendications 4-6, dans lequel le sel de métal alcalino-terreux est transformé en l'acide libre.

8. Composé cristallin qui est un sel de l'acide (6S)-5-méthyltétrahydrofolique avec au moins une base organique choisie dans le groupe constitué de 1-phényléthylamine, 1-p-nitrophényléthylamine, 1-p-chlorophényléthylamine, 1-p-méthylphényléthylamine, 1-p-méthoxyphényléthylamine, 1-(1-naphtyl)éthylamine, 1-(2-naphtyl)éthylamine, dans l'une ou l'autre configuration (R,S)- ou (R)- ou (S)-.

9. Composé selon la revendication précédente, qui est choisi dans le groupe constitué de (R, S)-1-phényléthylamine 5-méthyl-(6S)-tétrahydrofolate ; (R)-1-phényléthylamine 5- méthyl-(6S)-tétrahydrofolate ; (S)-1-phényléthylamine 5- méthyl- (6S) -tétrahydrofolate.

10. Utilisation du composé selon l'une quelconque des revendications 8 à 9, pour la séparation d'un mélange de diastéréoisomères de l'acide (6R,S)-5-acide méthyltétrahydrofolique ou d'un sel de métal alcalin de celui-ci comprenant la réaction du mélange dans de l'eau avec au moins une base organique sélectionnée parmi le groupe constitué de 1-phényléthylamine, 1-p-nitrophényléthylamine, 1-p-chlorophényléthylamine, 1-p-méthylphényléthylamine, 1-p-méthoxyphényléthylamine, 1-(1-naphtyl)éthylamine, 1-(2-naphtyl)éthylamine, dans l'une ou l'autre configuration (R,S)- ou (R)- ou (S)-, de manière à obtenir le sel d'amine correspondant, et la récupération d'un diastéréoisomère, ce dernier étant le diastéréoisomère (6S) naturel correspondant sensiblement pur.
